(19) **Europäisches Patentamt / European Patent Office / Office européen des brevets**

(11) **EP 1 500 397 B1**

(12) **EUROPEAN PATENT SPECIFICATION**

(45) Date of publication and mention
of the grant of the patent:
**28.03.2007  Bulletin 2007/13**

(51) Int Cl.:
**A61K 33/44** (2006.01)      **A61P 1/16** (2006.01)
**A61P 13/12** (2006.01)      **A61P 39/02** (2006.01)

(21) Application number: **03770095.2**

(22) Date of filing: **31.10.2003**

(86) International application number:
**PCT/JP2003/014012**

(87) International publication number:
**WO 2004/039381 (13.05.2004 Gazette 2004/20)**

(54) **ADSORBENTS FOR ORAL ADMINISTRATION, REMEDIES OR PREVENTIVES FOR KIDNEY DISEASES AND REMEDIES OR PREVENTIVES FOR LIVER DISEASES**

ADSORPTIONSMITTEL FÜR DIE ORALE VERABREICHUNG, MITTEL ZUR BEHANDLUNG ODER PRÄVENTION VON NIERENERKRANKUNGEN UND MITTEL ZUR BEHANDLUNG ODER PRÄVENTION VON LEBERERKRANKUNGEN

ADSORBANTS POUR ADMINISTRATION ORALE, REMEDES PREVENTIFS OU CURATIFS CONTRE LES MALADIES DES REINS ET REMEDES PREVENTIFS OU CURATIFS CONTRE LES MALADIES DU FOIE

(84) Designated Contracting States:
**AT BE BG CH CY CZ DE DK EE ES FI FR GB GR HU IE IT LI LU MC NL PT RO SE SI SK TR**

(30) Priority: **01.11.2002  JP 2002320253**

(43) Date of publication of application:
**26.01.2005  Bulletin 2005/04**

(73) Proprietor: **Kureha Corporation**
**Tokyo 103-8552 (JP)**

(72) Inventors:
• **SONOBE,N.**
**Nishiki Research Lab., Kureha Chemical**
**Iwaki-shi, Fukushima 974-8686 (JP)**
• **MORIMOTO,S**
**Development Department, Kureha Chemical**
**Shinjuku-ku, Tokyo 169-8503 (JP)**
• **YOSHIHARA, H.**
**Carbon Products Dep.,Kureha Chemical**
**1-chome**
**Chuo-ku, Toky (JP)**
• **HANATSUKA, Hiroyuki**
**Nishiki Plant, Kureha Chemical**
**Iwaki-shi, Fukushima 974-8686 (JP)**
• **ARAKAWA, Makoto**
**Nishiki Plant, Kureha Chemical**
**Iwaki-shi, Fukushima 974-8686 (JP)**

(74) Representative: **Minderop, Ralph H. et al**
**COHAUSZ & FLORACK**
**Patent- und Rechtsanwälte**
**Bleichstrasse 14**
**40211 Düsseldorf (DE)**

(56) References cited:
**EP-A- 1 440 692**      **EP-A1- 0 029 715**
**EP-A1- 0 595 715**      **EP-A1- 1 249 241**
**EP-A2- 0 711 561**      **GB-A- 2 053 176**
**JP-A- 11 292 770**      **JP-A- 11 292 771**
**JP-A- 58 213 613**

• **YANG J-B ET AL: "Preparation and properties of phenolic resin-based activated carbon spheres with controlled pore size distribution" CARBON, XX, XX, vol. 40, no. 6, May 2002 (2002-05), pages 911-916, XP004346745 ISSN: 0008-6223**
• **CHEMICAL ABSTRACTS, vol. 97, no. 22, 1982, Columbus, Ohio, US; abstract no.: 188342, page 394 XP002314268 & JP 82 136455 A (SUMITOMO BAKELITE) 23 August 1982 (1982-08-23)**
• **CHEMICAL ABSTRACTS, vol. 94, no. 22, 1981, Columbus, Ohio, US; abstract no.: 180725, page 384 XP002314269 & JP 08 128766 A (SUMITOMO BAKELITE) 2 March 1981 (1981-03-02)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

TECHNICAL FIELD

The present invention relates to an adsorbent for oral administration comprising a spherical activated carbon having a specific pore structure, and an adsorbent for oral administration comprising a surface-modified spherical activated carbon prepared by oxidizing and reducing the spherical activated carbon and having a similar specific pore structure. Further, the present invention relates to an agent for treating or preventing a renal or liver disease, comprising the adsorbent for oral administration as an effective component.

The adsorbent for oral administration, according to the present invention, exhibits a selective adsorbability, that is, a high adsorbability of harmful toxins, despite a low adsorbability of useful components such as digestive enzymes in a body. Further, the adsorbent has a specific pore structure, and thus, has a greatly improved selective adsorbability in comparison with that of a conventional adsorbent for oral administration. Therefore, the adsorbent for oral administration, according to the present invention, is effective for the treatment of a patient suffering from a liver or renal disease.

BACKGROUND ART

In patients suffering with a lack of a renal function or a liver function, harmful toxic substances are accumulated or formed in bodies, such as blood, with a progress of a disorder of the organ functions, and thus an encephalopathia occurs, such as a disturbance of consciousness or uremia. Yearly, there is a growing number of such patients, and therefore, the development of an organ-substitute apparatus or medicament having a function to remove toxic substances from bodies, in place of such defective organs, has become a serious problem. A method for removing toxic substances by hemodialysis as an artificial kidney is prevalent. Nevertheless, the hemodialysis-based artificial kidney requires a special apparatus, and thus, a skilled specialist is required from a safe operation standpoint. Further, blood must be taken from a patient's body, and thus, there are disadvantages in that patients must bear high physical, mental and economic burdens. Accordingly, hemodialysis is not satisfactory.

Recently, as a means of remedying the above disadvantages, an oral adsorbent which can be orally administered and cure a disorder of renal and liver functions has received considerable attention. Specifically, an adsorbent disclosed in Japanese Examined Patent Publication (Kokoku) No. 62-11611 comprises a porous spherical carbonaceous substance having particular functional groups (hereinafter referred to as a surface-modified spherical activated carbon); having a high safety factor and stable to a body; and having a useful selective adsorbability, that is, an excellent adsorbability of harmful substances in the presence of a bile acid in an intestine, and a low adsorbability of useful substances such as digestive enzymes in the intestine. For these reasons, the oral adsorbent is widely and clinically used for a patient suffering from a disorder of a liver or renal function, as an adsorbent having few side effects such as constipation. The above adsorbent disclosed in Japanese Examined Patent Publication (Kokoku) No. 62-11611 was prepared by forming a spherical activated carbon from a pitch such as a petroleum pitch as a carbon source, and then carrying out an oxidizing treatment and a reducing treatment. EP 0 711 561 A2 discloses the use of an activated spherical carbon in a preparation of an agent for reducing nephrotoxicity caused by a platinum complex compound. All preparations disclosed are obtained from Pitch. But a listing in the description mentions synthetic organic high polymers as one of possible further raw materials to obtain activated carbon, too.

DISCLOSURE OF THE INVENTION

The inventors of the present invention engaged in intensive research to develop an adsorbent for oral administration exhibiting a greater selective adsorbability than that of the above-mentioned oral adsorbent comprising the conventional porous spherical carbonaceous substance prepared by forming a spherical activated carbon from a pitch and oxidizing and reducing the activated carbon, and surprisingly, found that a spherical activated carbon prepared from a thermosetting resin as a carbon source, even without the oxidizing and reducing treatments, exhibits an excellent selective adsorbability; that is, on one hand, an excellent adsorbability of β-aminoisobutyric acid which is one of the uremic substances in a body, and on the other hand, a low adsorbability of useful substances, for example, digestive enzymes, such as α-amylase, and that a level of the selective adsorbability thereof is superior to that of the adsorbent disclosed in Japanese Examined Patent Publication (Kokoku) No. 62-11611. Because the above-mentioned spherical activated carbon prepared from the thermosetting resin as a carbon source exhibits an excellent adsorbability of β-aminoisobutyric acid, it is presumed that the above-mentioned spherical activated carbon has an excellent adsorbability of other toxic substances having a molecular weight similar to that of β-aminoisobutyric acid, for example, octopamine or α-aminobutyric acid, or dimethylamine, aspartic acid, or arginine which is a toxic substance or a precursor thereof in a renal disease, or other water-soluble basic or ampholytic substances.

It was thought that the conventional porous spherical carbonaceous substance, that is, the surface-modified spherical activated carbon used for the adsorbent disclosed in Japanese Examined Patent Publication (Kokoku) No. 62-11611, began to exhibit the selective adsorbability as above, after functional groups were introduced by the oxidizing and reducing treatments of the spherical activated carbon prepared from a pitch. Therefore, it is surprising that the spherical activated carbon prior to the oxidizing and reducing treatments exhibits a selective adsorbability, and the adsorbability per se is superior to that of the conventional adsorbent for oral administration.

Further, the present inventors found that the useful selective adsorbability; that is, on one hand, an excellent adsorbability of β-aminoisobutyric acid which is one of the uremic substances in a body, and on the other hand, a low adsorbability of useful substances, for example, digestive enzymes, such as α-amylase, is improved in a surface-modified spherical activated carbon prepared by oxidizing and reducing the above spherical activated carbon, in comparison with the adsorbent disclosed in Japanese Examined Patent Publication (Kokoku) No. 62-11611. Therefore, it is presumed that the surface-modified spherical activated carbon has a greater adsorbability of other toxic substances having a molecular weight similar to that of β-aminoisobutyric acid, for example, octopamine or α-aminobutyric acid, or dimethylamine, aspartic acid, or arginine which is a toxic substance or a precursor thereof in a renal disease, or other water-soluble basic or ampholytic substances.

The present invention is based on the above findings.

Accordingly, the present invention relates to an adsorbent for oral administration, characterized by comprising a spherical activated carbon prepared from a thermosetting resin as a carbon source, wherein a diameter is 0.01 to 1 mm, and a specific surface area determined by Langmuir's adsorption equation is 1000 $m^2$/g or more.

The present invention also relates to an adsorbent for oral administration, characterized by comprising a surface-modified spherical activated carbon prepared from a thermosetting resin as a carbon source, wherein a diameter is 0.01 to 1 mm, a specific surface area determined by Langmuir's adsorption equation is 1000 $m^2$/g or more, a total amount of acidic groups is 0.40 to 1.00 meq/g, and a total amount of basic groups is 0.40 to 1.10 meq/g.

Further, the present invention also relates to an agent for treating or preventing a renal or liver disease, comprising the above adsorbent for oral administration as an effective component.

BRIEF DESCRIPTION OF THE DRAWINGS

Figure 1 is a micrograph (magnification: x50) illustrating a surface structure of a surface-modified spherical activated carbon of the present invention obtained by a scanning electron microscope.

Figure 2 is a micrograph (magnification: x200) illustrating a cross sectional structure of a surface-modified spherical activated carbon of the present invention obtained by a scanning electron microscope.

Figure 3 is a micrograph (magnification: x50) illustrating a surface structure of a surface-modified spherical activated carbon of the prior art obtained by a scanning electron microscope.

Figure 4 is a micrograph (magnification: x200) illustrating a cross sectional structure of a surface-modified spherical activated carbon of the prior art obtained by a scanning electron microscope.

Figure 5 is a graph showing the results of the investigation of the effect of the adsorbent for oral administration of the present invention on serum creatinine.

Figure 6 is a graph showing the results of the investigation of the effect of the adsorbent for oral administration of the present invention on blood urea nitrogen.

Figure 7 is a graph showing the results of the investigation of the effect of the adsorbent for oral administration of the present invention on creatinine clearance.

Figure 8 is a graph showing the results of the investigation of the effect of the adsorbent for oral administration of the present invention on an amount of urine protein excreted.

Figure 9 is a graph showing the results of the investigation of the effect of the adsorbent for oral administration of the present invention on ICG (Indocyanine green).

Figure 10 is a graph showing the results of the investigation of the effect of the adsorbent for oral administration of the present invention on GOT (glutamic-oxaloacetic transaminase).

Figure 11 is a graph showing the results of the investigation of the effect of the adsorbent for oral administration of the present invention on GPT (glutamic-pyruvic transaminase)..

BEST MODE FOR CARRYING OUT THE INVENTION

As explained, the spherical activated carbon or the surface-modified spherical activated carbon used as the adsorbent for oral administration of the present invention is characterized in that a thermosetting resin is used as a carbon source instead of a pitch used as a carbon source for the adsorbent for oral administration of prior art. The spherical activated carbon or the surface-modified spherical activated carbon used as the adsorbent for oral administration of the present invention can be prepared by carrying out procedures substantially same as those in a conventional method using pitch, except for the above characteristic feature.

The spherical activated carbon or the surface-modified spherical activated carbon used as the adsorbent for oral administration of the present invention can be prepared by, for example, the following methods.

A spherical material of a thermosetting resin is initially activated at 700 to 1000°C in a reactive gas stream with carbon (for example, steam or carbon dioxide gas) to obtain the spherical activated carbon used as the adsorbent for oral administration of the present invention. The term spherical "activated carbon" as used herein means a porous product prepared by a heat-treatment of a carbon precursor such as a spherical thermosetting resin, and subsequent activation, and having a spherical shape and a specific surface area of 100 $m^2/g$ or more, preferably 1000 $m^2/g$ or more in the present invention.

If the spherical material of a thermosetting resin is softened by the heat-treatment and changed to an aspheric shape, or fused together by the heat-treatment, the softening can be inhibited by an oxidation at 150°C to 400°C in an atmosphere containing oxygen as a treatment imparting infusibility, before the activation as above.

Further, if many pyrolysis gases or the like are generated by the heat-treatment of the spherical thermosetting resin, pyrolysis products may be removed in advance by accordingly carrying out a pre-calcination, prior to the treatment imparting infusibility.

The inventors of the present invention found that a more excellent selective adsorbability can be obtained when the spherical activated carbon of the present invention has a total amount of basic groups of 0.40 meq/g or more. The total amount of basic groups is more preferably 0.6 meq/g or more, most preferably 0.7 meq/g or more.

In order to further improve the selective adsorbability of the spherical activated carbon of the present invention, the resulting spherical activated carbon is subsequently oxidized at 300 to 800°C, preferably 320 to 600°C, in an atmosphere containing 0.1 to 50 vol%, preferably 1 to 30 vol%, particularly preferably 3 to 20 vol% of oxygen, and then reduced by a heat-reaction at 800 to 1200°C, preferably 800 to 1000°C, in an atmosphere of non-oxidative gas, to thereby obtain the surface-modified spherical activated carbon used as the adsorbent for oral administration according to the present invention. The term "surface-modified spherical activated carbon" as used herein means a porous product prepared by the oxidizing and reducing treatments of the spherical activated carbon as above, wherein acidic and basic sites are added in a well-balanced manner on the surface of the spherical activated carbon to thereby improve an adsorbability of harmful substances in an intestine.

A particle diameter of the spherical product of a thermosetting resin used as a starting material is preferably about 0.02 to 1.5 mm.

It is important for the thermosetting resin used as the starting material that a spherical product can be formed, and it is not fused or softened, or the shape is not changed, by a heat-treatment at a temperature of 500°C or less. A thermosetting resin which can avoid a fusion oxidation by the treatment imparting infusibility, such as an oxidation treatment, can be used.

A thermosetting resin which can obtain a high carbonization yield by a heat-treatment is preferable as a starting material. If the carbonization yield is low, a strength of the spherical activated carbon becomes low. Further, undesirable pores are formed and a bulk density of the spherical activated carbon is lowered, and thus, a specific surface area per volume is lowered. Therefore, a volume to be orally administered is increased, and thus, a problem arises in that an oral administration becomes difficult. Accordingly, a thermosetting resin having a higher carbonization yield is preferable. A yield by a heat-treatment at 800°C in an atmosphere of non-oxidative gas is preferably 40 % by weight or more, more preferably 45 % by weight or more.

The thermosetting resin used as a starting material may be, for example, a phenolic resin, such as a novolak phenolic resin, a resol phenolic resin, a novolak alkylphenolic resin, or a resol alkylphenolic resin, or a furan resin, a urea resin, a melamine resin, or an epoxy resin. A copolymer of divinylbenzene and styrene, acrylonitrile, acrylic acid, or methacrylic acid may be used as the thermosetting rein.

Further, an ion-exchange resin may be used as the thermosetting resin. Generally, an ion-exchange resin comprises a copolymer of divinylbenzene and styrene, acrylonitrile, acrylic acid, or methacrylic acid, that is, a thermosetting resin, and essentially has a structure wherein ion-exchange groups are bonded to a copolymer matrix having a three-dimensional network skeleton. The ion-exchange resin is generally classified, with respect to the kinds of ion-exchange groups, into a strongly acidic ion-exchange resin having sulfonic acid groups, a weakly acidic ion-exchange resin having carboxylic or sulfonic acid groups, a strongly basic ion-exchange resin having quaternary ammonium salts, and a weakly basic ion-exchange resin having primary or tertiary amines. In addition, so-called hybrid ion-exchange resin having both acidic and basic ion-exchange groups is included as a special ion-exchange resin. In the present invention, all of the above ion-exchange resins may be used as a starting material, but a phenolic resin is preferably used.

The spherical activated carbon or the surface-modified spherical activated carbon used as the adsorbent for oral administration of the present invention is produced by, for example, the above methods using the thermosetting resin as a starting material, and has a diameter of 0.01 to 1 mm. If the diameter of the spherical activated carbon or the surface-modified spherical activated carbon is less than 0.01 mm, an exterior surface area of the spherical activated carbon or the surface-modified spherical activated carbon is increased, and useful substances such as digestive enzymes are easily adsorbed. That is unfavorable. When the diameter is more than 1 mm, a diffusion distance of toxic substances into the inside of the spherical activated carbon or the surface-modified spherical activated carbon is increased, and an adsorption rate is lowered. That, too, is unfavorable. The diameter is preferably 0.02 to 0.8 mm. The expression that "a diameter is Dl to Du" as used herein means that a screen passing percentage (%) in a range of a screen opening Dl to Du is 90% or more in a particle-sizes accumulating standard curve prepared in accordance with JIS K 1474, as mentioned below in relation with a method for determining an average particle diameter.

In the spherical activated carbon or the surface-modified spherical activated carbon used as the adsorbent for oral administration of the present invention, a specific surface area (referred to as "SSA" hereinafter) determined by Langmuir's adsorption equation is 1000 $m^2/g$ or more. When the spherical activated carbon or the surface-modified spherical activated carbon has an SSA of less than 1000 $m^2/g$, an adsorbability of toxic substances is unfavorably lowered. The SSA is preferably 1000 $m^2/g$ or more. The upper limit of the SSA is not particularly limited, but the SSA is preferably 3000 $m^2/g$ or less in view of a bulk density and strength.

In the spherical activated carbon or the surface-modified spherical activated carbon used as the adsorbent for oral administration of the present invention, a pore volume within a scope of specific pore diameters is not particularly limited. For example, the above-mentioned Japanese Examined Patent Publication (Kokoku) No. 62-11611 discloses an adsorbent comprising a surface-modified spherical activated carbon wherein a volume of voids having a pore radius of 100 to 75000 angstrom, that is, a volume of pores having a diameter of 20 to 15000 nm, is 0.1 to 1 mL/g. However, in the spherical activated carbon or the surface-modified spherical activated carbon used as the adsorbent for oral administration of the present invention, a volume of pores having a diameter of 20 to 15000 nm may be 0.1 to 1 mL/g, or 0.1 mL/g or less. When a volume of pores having a diameter of 20 to 1000 nm is more than 1 mL/g, an adsorbed amount of useful substances, such as digestive enzymes, may be increased. Therefore, a volume of pores having a diameter of 20 to 1000 nm is preferably 1 mL/g or less.

In the spherical activated carbon or the surface-modified spherical activated carbon used as the adsorbent for oral administration of the present invention, a volume of pores having a diameter of 7.5 to 15000 nm is preferably less than 0.25 mL/g, more preferably 0.2 mL/g or less, as a more excellent selective adsorbability is thus obtained.

In a constitution of functional groups of the surface-modified spherical activated carbon, that is, the product prepared by oxidizing and reducing the spherical activated carbon, which is used as the adsorbent for oral administration of the present invention, a total amount of acidic groups is 0.40 to 1.00 meq/g, and a total amount of basic groups is 0.40 to 1.10 meq/g. When the constitution of functional groups satisfies the condition that a total amount of acidic groups is 0.40 to 1.00 meq/g, and a total amount of basic groups is 0.40 to 1.00 meq/g, the selective adsorbability is improved, and particularly, the adsorbability of harmful substances is favorably enhanced. In the constitution of functional groups, a total amount of acidic groups is preferably 0.40 to 0.90 meq/g, and a total amount of basic groups is preferably 0.40 to 1.00 meq/g.

When the adsorbent of the present invention is used as an agent for treating or preventing a liver or renal disease, a preferable functional-groups constitution is that the total amount of acidic groups is 0.40 to 1.00 meq/g, the total amount of basic groups is 0.40 to 1.10 meq/g, a phenolic hydroxyl group is 0.20 to 0.70 meq/g, and a carboxyl group is 0.15 meq/g or less, and a ratio (a/b) of the total amount of acidic groups (a) to the total amount of basic groups (b) is 0.40 to 2.5, and a relation [(b+c)-d] between the total amount of basic groups (b), the phenolic hydroxyl group (c), and the carboxyl group (d) is 0.60 or more.

Properties of the spherical activated carbon or the surface-modified spherical activated carbon used as the adsorbent for oral administration of the present invention, namely, the average particle diameter, the specific surface area, the pore volume, the total amount of acidic groups, and the total amount of basic groups are measured by the following methods.

(1) An average particle diameter

A particle-sizes accumulating standard curve is prepared in accordance with JIS K 1474 for the spherical activated carbon or the surface-modified spherical activated carbon. The average particle diameter is determined from a screen opening (mm) at an intersection point with a line that is horizontal to an abscissa axis and starts from an intersection point in the particle-sizes accumulating standard curve with a perpendicular line from a 50% point of the abscissa axis.

(2) A specific surface area

An amount of gas adsorbed is measured by a specific surface area measuring apparatus (for example, ASAP2010 manufactured by MICROMERITICS) in accordance with a gas adsorbing method for the spherical activated carbon sample or the surface-modified spherical activated carbon sample, and a specific surface area can be calculated by Langmuir's adsorption equation. More particularly, the spherical activated carbon or the surface-modified spherical activated carbon is charged as a sample in a sample tube, and dried under reduced pressure at 300°C. Thereafter, a weight of dried sample is measured. Then, the test tube is cooled to -196°C, and nitrogen is introduced into the test tube, whereby nitrogen is adsorbed to the spherical activated carbon sample or the surface-modified spherical activated carbon sample. A relation of a nitrogen partial pressure and an adsorbed amount (absorption-isotherm line) is measured.

Langmuir's plotting is carried out, given that a relative pressure of nitrogen is p, and an adsorbed amount at that time is $v(cm^3/g$ STP). That is, the plotting in a range wherein p is 0.05 to 0.3 is carried out, in the field wherein a longitudinal axis is $p/v$, and an abscissa axis is p. Given that the gradient at that time is $b(g/cm^3)$, a specific surface area S (unit = $m^2/g$) can be calculated from the equation:

$$S = \frac{MA \times \left(6.02 \times 10^{23}\right)}{22414 \times 10^{18} \times b}$$

wherein MA denotes a cross-sectional area of a nitrogen molecule, and is $0.162\ nm^2$.

(3) A pore volume by a mercury press-injection method

The pore volume can be measured by a mercury porosimeter (for example, AUTOPORE 9200 manufactured by MICROMERITICS). The spherical activated carbon or the surface-modified spherical activated carbon is charged as a sample in a sample vessel, and degassed under a pressure of 2.67Pa or less for 30 minutes. Then, mercury is introduced into the sample vessel, a pressure applied is gradually increased (maximum pressure = 414 MPa) to force the mercury into the micropores in the spherical activated carbon sample or the surface-modified spherical activated carbon sample. A pore volume distribution of the spherical activated carbon sample or the surface-modified spherical activated carbon sample is measured from a relationship between the pressure and an amount of forced mercury, by equations as mentioned below.

Specifically, a volume of mercury inserted into the spherical activated carbon sample or the surface-modified spherical activated carbon sample while a pressure applied is increased from a pressure (0.06 MPa) corresponding to a pore diameter of 22 $\mu$m to the maximum pressure (414 MPa) corresponding to a pore diameter of 3 nm is measured. A pore diameter can be calculated as follows. When mercury is forced into a cylindrical micropore having a diameter (D) by applying a pressure (P), a surface tension ($\gamma$) of mercury is balanced with a pressure acting on a section of the micropore, and thus, the following equation is held:

$$-\pi D\gamma\cos\theta = \pi(D/2)^2 \cdot P$$

wherein $\theta$ is a contact angle of mercury and a wall of the micropore. Therefore, the following equation:

$$D = (-4\gamma\cos\theta)/P$$

is held.

In the present specification, the relationship between the pressure (P) and the pore diameter (D) is calculated by the equation:

$$D = 1.27/P$$

given that a surface tension of mercury is 484 dyne/cm, a contact angle of mercury and carbon is 130°, a unit of the pressure P' is MPa, and a unit of the pore diameter D is $\mu$m. The volume of pores having a pore diameter of 20 to 1000 nm in the present invention corresponds to a volume of mercury inserted by applying a pressure increasing from 1.27 MPa to 63.5 MPa, and the volume of pores having a pore diameter of 7.5 to 15000 nm corresponds to a volume of mercury inserted by applying a pressure increasing from 0.085 MPa to 169 MPa.

(4) Total amount of acidic groups

The total amount of acidic groups is an amount of NaOH consumed, which may be determined by adding 1 g of the spherical activated carbon sample or the surface-modified spherical activated carbon sample, after being crushed to form particles having a size of 200 mesh or less, to 50 mL of a 0.05N NaOH solution; shaking the mixture for 48 hours; then filtering out the spherical activated carbon sample or the surface-modified spherical activated carbon sample; and titrating until neutralization.

(5) Total amount of basic groups

The total amount of basic groups is an amount of HCl consumed, which may be determined by adding 1 g of the spherical activated carbon sample or the surface-modified spherical activated carbon sample after being crushed to form particles having a size 200 mesh or less, to 50 mL of a 0.05N HCl solution; shaking the mixture for 24 hours; then filtering out the spherical activated carbon sample or the surface-modified spherical activated carbon sample; and titrating until neutralization.

As shown in Examples mentioned as below, the spherical activated carbon or the surface-modified spherical activated carbon used as the adsorbent for oral administration of the present invention exhibits an excellent selective adsorbability, that is, an excellent adsorbability of exacerbation factors of liver diseases or harmful substances of renal diseases, but a lower adsorbability of useful substances such as digestive enzymes, and therefore, may be used as an adsorbent for oral administration for treating or preventing a renal disease or a liver disease.

As the renal disease, there may be mentioned, for example, chronic renal failure, acute renal failure, chronic pyelonephritis, acute pyelonephritis, chronic nephritis, acute nephritic syndrome, acute progressive nephritic syndrome, chronic nephritic syndrome, nephrotic syndrome, nephrosclerosis, interstitial nephritis, tubulopathy, lipoid nephrosis, diabetic nephropathy, renovascular hypertension, or hypertension syndrome, or secondary renal diseases caused by these primary diseases, or a light renal failure before a dialysis therapy, and may be used in an improvement of a light renal failure before a dialysis therapy or a disease condition for a patient during a dialysis therapy (see "Clinical Nephrology", Asakura-shoten, Nishio Honda, Kenkichi Koiso, and Kiyoshi Kurokawa, 1990; and "Nephrology" Igaku-shoin, Teruo Omae and Sei Fujimi, ed., 1981).

As the liver disease, there may be mentioned, for example, fulminant hepatitis, chronic hepatitis, viral hepatitis, alcoholic hepatitis, hepatic fibrosis, liver cirrhosis, hepatic cancer, autoimmune hepatitis, drug allergic hepatopathy, primary biliary cirrhosis, tremor, encephalopathia, dysbolism, or dysfunction. Further, the porous spherical carbonaceous substance can be used in a treatment of a disease caused by toxic substances in a body, such as psychosis.

Therefore, when the adsorbent for oral administration is used as an agent for treating or preventing a renal disease, it contains the spherical activated carbon or the surface-modified spherical activated carbon as an effective component. When the adsorbent for oral administration according to the present invention is used as an agent for a treatment of a liver or renal disease, a dosage thereof depends on the subject (human or other animal), age, individual differences, disease conditions, and so on. Therefore, in some cases, a dosage outside of the following dosage may be appropriate, but in general, the oral dosage in the case of a human is usually 1 to 20 g of the adsorbent per day, wherein the daily dosage may be divided into three to four portions. The dosage may appropriately vary with the disease conditions. The formulation may be administered in any form, such as powders, granules, tablets, sugar-coated tablets, capsules, suspensions, sticks, divided packages, or emulsions. In the case of capsules, the usual gelatin capsules, or if necessary, enteric capsules may be used. In the case of tablets, the formulations must be broken into the original fine particles inside the body. The adsorbent may be used as a mixture with an electrolyte-controlling agent, such as an aluminum gel or Kayexalate.

EXAMPLES

The present invention now will be further illustrated by, but is by no means limited to, the following Examples.

In the following Examples, an adsorption test of α-amylase and an adsorption test of DL-β-aminoisobutyric acid were carried out in accordance with the following methods, and the selective adsorption rate was calculated by the following method.

(1) Adsorption test of α-amylase

The spherical activated carbon sample or the surface-modified spherical activated carbon sample was dried, and 0.125 g of the dried sample was accurately weighed and charged into a conical flask equipped with a ground-in stopper. On the other hand, 0.100 g of α-amylase (liquefied type) was accurately weighed and dissolved by adding a phosphate buffer (pH 7.4) to prepare a stock solution having an accurate volume of 1000 mL. The stock solution in an accurate amount of 50 mL was charged to the conical flask equipped with a ground-in stopper. The flask was shaken at 37±1 °C for 3 hours. The product in the flask was filtered with suction through a 0.65 μm membrane filter. A first filtrate (about 20 mL) was discarded, and a subsequent filtrate (about 10 mL) was taken as a sample solution.

Further, the same procedures were repeated except that only a phosphate buffer (pH 7.4) was used, to obtain a filtrate as a correction solution. The sample solution and the correction solution were analyzed by an absorptiometeric analysis, using a phosphate buffer (pH 7.4) as a control. The absorbance at a wavelength of 282 nm was measured. A difference between the absorbance of the sample solution and the absorbance of the correction solution was taken as a test absorbance.

A standard curve was prepared by adding the α-amylase stock solution in an accurate amount of 0 mL, 25 mL, 50 mL, 75 mL, or 100 mL to a measuring flask, adding a phosphate buffer (pH 7.4) to 100 mL, and measuring an absorbance at a wave length of 282 nm. From the test absorbance and the standard curve, an amount (mg/dL) of α-amylase remaining in the solution was calculated.

To measure a dependence on an amount of the spherical activated carbon sample or the surface-modified spherical activated carbon sample, the same procedures were repeated except that an amount of the spherical activated carbon sample or the surface-modified spherical activated carbon sample used was 0.500 g, and the test absorbance was measured and the amount of α-amylase remaining in the solution was calculated as above.

(2) Adsorption test of DL-β-aminoisobutyric acid

The spherical activated carbon sample or the surface-modified spherical activated carbon sample was dried, and 2.500 g of the dried sample was accurately weighed and charged into a conical flask equipped with a ground-in stopper. On the other hand, 0.100 g of DL-β-aminoisobutyric acid was accurately weighed and dissolved by adding a phosphate buffer (pH 7.4) to prepare a stock solution having an accurate volume of 1000 mL. The stock solution in an accurate amount of 50 mL was charged to the conical flask equipped with a ground-in stopper. The flask was shaken at 37±1 °C for 3 hours. The product in the flask was filtered with suction through a 0.65 μm membrane filter. A first filtrate (about 20 mL) was discarded, and a subsequent filtrate (about 10 mL) was taken as a sample solution.

Then, 0.1 mL of the sample solution was accurately weighed and charged in a test tube. A phosphate buffer (pH 8.0) was added in an accurate amount of 5 mL thereto, and the whole was mixed. Thereafter, a solution prepared by dissolving 0.100 g of fluorescamine in 100 mL of acetone (for a non-aqueous titration) was added in an accurate amount of 1 mL, and the whole was mixed and allowed to stand for 15 minutes. The resulting solution was analyzed by fluorometry, and the fluorescence was measured at an exciting wavelength of 390 nm and a fluorescent wavelength of 475 nm.

A standard curve was prepared by producing 100 mL of a mixture of 0 mL, 15 mL, 50 mL, 75 mL, and 100 mL of the DL-β-aminoisobutyric acid stock solution and the balance of a phosphate buffer (pH 7.4), stirring and filtering the mixture, charging the resulting filtrate in an accurate amount of 0.1 mL to a test tube, adding a phosphate buffer (pH 8.0) in an accurate amount of 5 mL, mixing the whole, adding a solution (an accurate amount: 1 mL) prepared by dissolving 0.100 g of fluorescamine in 100 mL of acetone (for a non-aqueous titration), mixing the whole, allowing to stand for 15 minutes, analyzing the resulting solution by fluorometry, and measuring the fluorescence at an exciting wavelength of 390 nm and a fluorescent wavelength of 475 nm. Finally, an amount (mg/dL) of DL-β-aminoisobutyric acid remaining in the solution was calculated, using the standard curve.

To measure a dependence on an amount of the spherical activated carbon sample or the surface-modified spherical activated carbon sample, the same procedures were repeated except that an amount of the spherical activated carbon sample or the surface-modified spherical activated carbon sample used was 0.500 g, and the test fluorescence was measured and the amount of DL-β-aminoisobutyric acid remaining in the solution was calculated as above.

(3) The selective adsorption rate

The selective adsorption rate was calculated from an amount of α-amylase remaining in the solution in the adsorption test of α-amylase wherein an amount of the spherical activated carbon sample used or the surface-modified spherical activated carbon sample used was 0.500 g, and an amount of DL-β-aminoisobutyric acid remaining in the solution in the adsorption test of DL-β-aminoisobutyric acid, wherein an amount of the spherical activated carbon sample used or the surface-modified spherical activated carbon sample used was 0.500 g, using the equation:

$$A = (10-Tr)/(10-Ur)$$

wherein A denotes the selective adsorption rate, and Tr denotes an amount of DL-β-aminoisobutyric acid remaining in the solution, and Ur denotes an amount of $\alpha$-amylase remaining in the solution.

## EXAMPLE 1

Spherical phenolic resin (particle diameter = 10 to 700 $\mu$m: trade name = High functional true spherical resin "Maririn" HF500 type; Gun Ei Chemical Industry Co., Ltd.) was sieved through a screen having an opening size of 250 $\mu$m, to remove fine powders. Then, 150 g of the resulting spherical phenolic resin was charged into a vertical reaction quartz tube having a grating, heated to 350°C over 1.5 hours under a nitrogen gas stream, and further heated to 900°C over 6 hours, and maintained at 900°C for 1 hour to obtain 68.1 g of a spherical carbonaceous material. Thereafter, the product was activated at 900°C at an atmosphere of a gas mixture of nitrogen gas (3 NL/min) and steam (2.5 NL/min). When a packing density of the spherical activated carbon was lowered to 0.5 mL/g, the activation was ceased to obtain 29.9g of the spherical activated carbon (yield = 19.9% by weight).
The properties of the resulting spherical activated carbon are listed in Tables 1 and 2.

## EXAMPLE 2

The procedure described in Example 1 was repeated, except that a spherical phenolic resin (particle diameter = 700 $\mu$m: trade name = Spherical cured phenolic resin ACS series PR-ACS-2-50C; Sumitomo Bakelite Co., Ltd.) was used instead of the spherical phenolic resin used in Example 1, i.e., the spherical phenolic resin manufactured by Gunei Kagaku K.K., to obtain the spherical activated carbon (yield = 26.5%).
The properties of the resulting spherical activated carbon are listed in Tables 1 and 2.

## EXAMPLE 3

The spherical activated carbon obtained in Example 1 was oxidized at 470°C for 3 hours and 15 minutes on a fluidized bed at an atmosphere of a gas mixture of nitrogen gas and oxygen gas (oxygen concentration = 18.5 vol%), and then, reduced at 900°C for 17 minutes on the fluidized bed at an atmosphere of nitrogen gas to obtain the surface-modified spherical activated carbon.
The properties of the resulting surface-modified spherical activated carbon are listed in Tables 1 and 2.

## EXAMPLE 4

The procedure described in Example 3 was repeated, except that the spherical activated carbon used in Example 2 was used as the starting material, to obtain the surface-modified spherical activated carbon.
The properties of the resulting surface-modified spherical activated carbon are listed in Tables 1 and 2.

## EXAMPLE 5

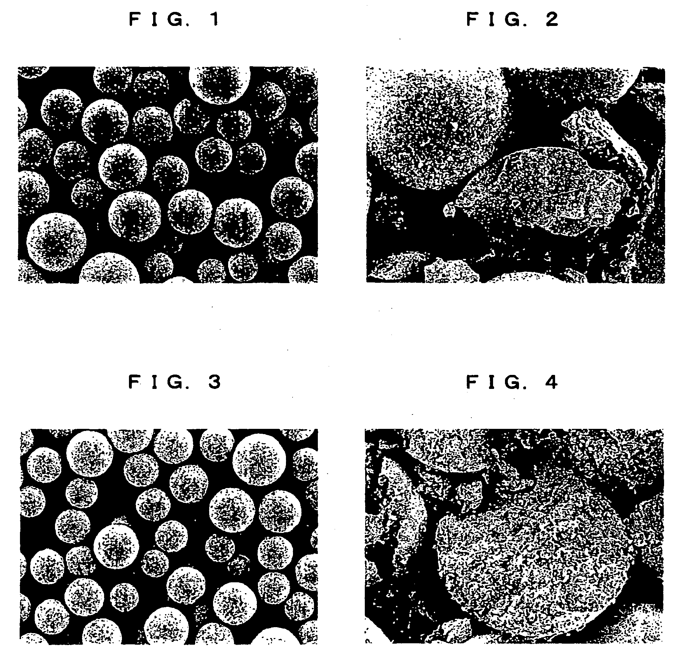

The procedure described in Example 3 was repeated, except that an ion-exchange resin (styrene based; effective diameter = 0.50 to 0.65 mm; trade name = Amberlite 15WET; Organo Corporation) was used instead of the phenolic resin, to obtain the surface-modified spherical activated carbon.
The properties of the resulting surface-modified spherical activated carbon are listed in Tables 1 and 2.
Further, a micrograph (magnification: x50) illustrating a surface structure of the resulting surface-modified spherical activated carbon obtained by a scanning electron microscope is shown in Figure 1, and a micrograph (magnification: x200) illustrating a cross sectional structure of the resulting surface-modified spherical activated carbon obtained by a scanning electron microscope is shown in Figure 2.

## COMPARATIVE EXAMPLE 1

Petroleum pitch (68 kg) (softening point = 210 °C; quinoline insoluble contents = not more than 1% by weight; ratio of hydrogen atoms/carbon atoms = 0.63) and naphthalene (32 kg) were charged into an autoclave (internal volume = 300 L) equipped with stirring fans, melted at 180°C, and mixed. The mixture was extruded at 80 to 90°C to form string-like shaped products. Then, the string-like shaped products were broken so that a ratio of a diameter to a length became about 1 to 2.
The resulting broken products were added to an aqueous solution prepared by dissolving 0.23% by weight of polyvinyl alcohol (saponification value = 88%) and heating to 93°C, and dispersed with stirring to be spheroidized. Then, the whole was cooled by replacing the polyvinyl alcohol aqueous solution with water, at 20°C for 3 hours, whereby the pitch was solidified and naphthalene crystals were precipitated, and a slurry of spherical shaped products of pitch was obtained.

After most of the water was removed by filtration, the naphthalene in the pitch was extracted and removed with n-hexane at an amount of about 6 times that of the spherical shaped products of pitch. The resulting porous spherical pitch was heated to 235°C by passing a heated air in a fluidized bed, and allowed to stand at 235°C for 1 hour, to thereby be oxidized, and a porous spherical oxidized pitch was obtained, which is non-fusible to heat. The resulting porous spherical oxidized pitch had an oxygen content of 14% by weight.

Thereafter, the resulting porous spherical oxidized pitch was activated in a fluidized bed at 900°C for 170 minutes by a nitrogen gas atmosphere containing 50% by volume of steam to obtain a spherical activated carbon. Further, the resulting spherical activated carbon was oxidized in the fluidized bed at 470°C for 195 minutes by a nitrogen-oxygen atmosphere containing 18.5% by volume of oxygen, and reduced in the fluidized bed at 900°C for 17 minutes by a nitrogen gas atmosphere, to obtain a surface-modified spherical activated carbon.

The properties of the resulting surface-modified spherical activated carbon are listed in Tables 1 and 2.

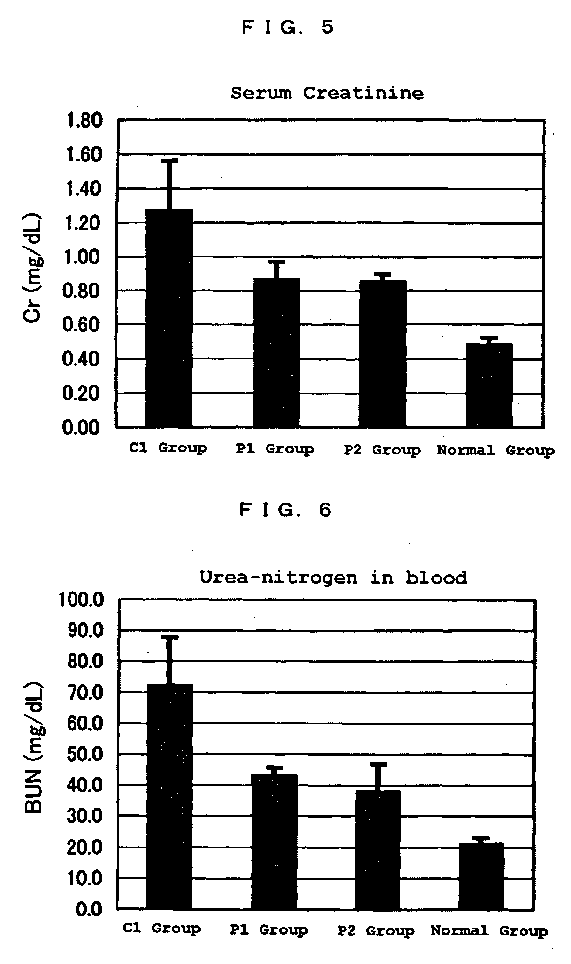

Further, a micrograph (magnification: x50) illustrating a surface structure of the resulting surface-modified spherical activated carbon obtained by a scanning electron microscope is shown in Figure 3, and a micrograph (magnification: x200) illustrating a cross sectional structure of the resulting surface-modified spherical activated carbon obtained by a scanning electron microscope is shown in Figure 4.

COMPARATIVE EXAMPLE 2

The procedure described in Comparative Example 1 was repeated, except that the oxidizing and reducing treatment of the spherical activated carbon were not carried out, to obtain the spherical activated carbon.

The properties of the resulting spherical activated carbon are listed in Tables 1 and 2.

Table 1

| | Raw material | SSA | | Hg pore volume | | Average particle diameter |
|---|---|---|---|---|---|---|
| | | Langmuir $m^2/g$ | BET $m^2/g$ | 20~1000nm | 7.5~15000nm | $\mu m$ |
| Example 1 | Phenolic resin | 2390 | 1860 | 0.0185 | 0.04 | 300 |
| Example 2 | Phenolic resin | 2100 | 1720 | 0.0272 | 0.06 | 430 |
| Example 3 | Phenolic resin | 2100 | 1670 | 0.0142 | 0.04 | 280 |
| Example | 4 Phenolic resin | 1930 | 1560 | 0.0185 | 0.06 | 410 |
| Example 5 | Ion-exchange resin | 1630 | 1250 | 0.2437 | 0.42 | 350 |
| Comparative Example 1 | Pitch | 2050 | 1540 | 0. 0750 | 0.11 | 350 |
| Comparative Example 2 | Pitch | 2100 | 1650 | 0.0850 | 0.15 | 350 |

The Hg pore volume in Table 1 was determined by a mercury press-injection method and corresponds to a volume of pores having a diameter of 20 to 1000 nm.

The SSA (BET) in Table 1 is a found value of a specific surface area listed as a reference, and determined by the following method.

As the method for determination of a specific surface area by Langmuir's adsorption equation, nitrogen is adsorbed to the spherical activated carbon sample or the surface-modified spherical activated carbon sample at -196°C, and a relation of a nitrogen partial pressure and an adsorbed amount (absorption isotherm) is measured.

BET plotting is carried out, given that a relative pressure of nitrogen is p, and an adsorbed amount at that time is v ($cm^3$/g STP). That is, the plotting in a range wherein p is 0.05 to 0.3 is carried out, in the field wherein a longitudinal axis is p/(v(1-p)), and an abscissa axis is p. From the gradient at that time of b (unit = $g/cm^3$), and an intercept of c (unit = $g/cm^3$), a specific surface area S (unit = $m^2$/g) can be calculated from the equation:

$$S = \frac{MA \times \left(6.02 \times 10^{23}\right)}{22414 \times 10^{18} \times (b+c)}$$

wherein MA denotes a cross-sectional area of a nitrogen molecule, and was 0.162 $nm^2$.

Table 2

| | Total amount of acidic groups | Total amount of basic groups | Amount of α-amylase remaining in solutions (mg/dL) | | Amount of DL-β-aminoisobutyric acid remaining in solutions (mg/dL) | | Selective adsorbability |
|---|---|---|---|---|---|---|---|
| | meq/g | meq/g | 0.125g | 0.50g | 0.50g | 2.50g | |
| Example 1 | 0.27 | 0.82 | 9.1 | 9.1 | 5.9 | 0.1 | 4.6 |
| Example 2 | 0.21 | 0.65 | 9.0 | 9.0 | 7.4 | 1.3 | 2.6 |
| Example 3 | 0.67 | 0.72 | 9.1 | 8.9 | 4.8 | 0.2 | 4.7 |
| Example 4 | 0.72 | 0.57 | 9.0 | 8.9 | 5.6 | 0.4 | 4.0 |
| Example 5 | 0.65 | 0.59 | 8.9 | 7.2 | 4.1 | 0.1 | 2.1 |
| Comparative Example 1 | 0.67 | 0.54 | 8.5 | 7.2 | 5.24 | 0.14 | 1.7 |
| Comparative Example 2 | 0.18 | 0.58 | 8.6 | 7.7 | 8.46 | 4.3 | 0.7 |

Test 1 for confirming pharmacological effects: Function to improve a renal disease

Renal failure model rats induced by subtotal nephrectomy of 3/4 kidney were used to carry out a test for confirming pharmacological effects on a renal failure by an administration of the adsorbent for oral administration of the present invention. The adsorbents prepared in Examples 1 and 3 according to the present invention were used as a sample. After six weeks from the induction to produce model rats, the rats were divided into a control group (6 rats; hereinafter referred to as a C1 group), a group to which the adsorbent prepared in Example 1 was administered (6 rats; hereinafter referred to as a P1 group), and a group to which the adsorbent prepared in Example 3 was administered (6 rats; hereinafter referred to as a P2 group), so that there was no major imbalance therebetween.

A powdery feed was administered to the rats of the groups. An amount of the feed given to the rats of the groups was determined on the basis of an average amount of feed taken by the rats of the C1 group for 2 or 3 days. A mixed feed containing 5% by weight of the adsorbent for oral administration in the same powdery feed as that administered to the C1 group was administered to the rats of the P1 and P2 groups. After 8 weeks from the beginning of the administration of the adsorbents for oral administration, serum creatinine, urea-nitrogen, urinary creatinine, creatinine clearance, and an amount of protein excreted were measured. Further, a same test was carried out for six normal rats in which subtotal nephrectomy was not conducted (normal group).

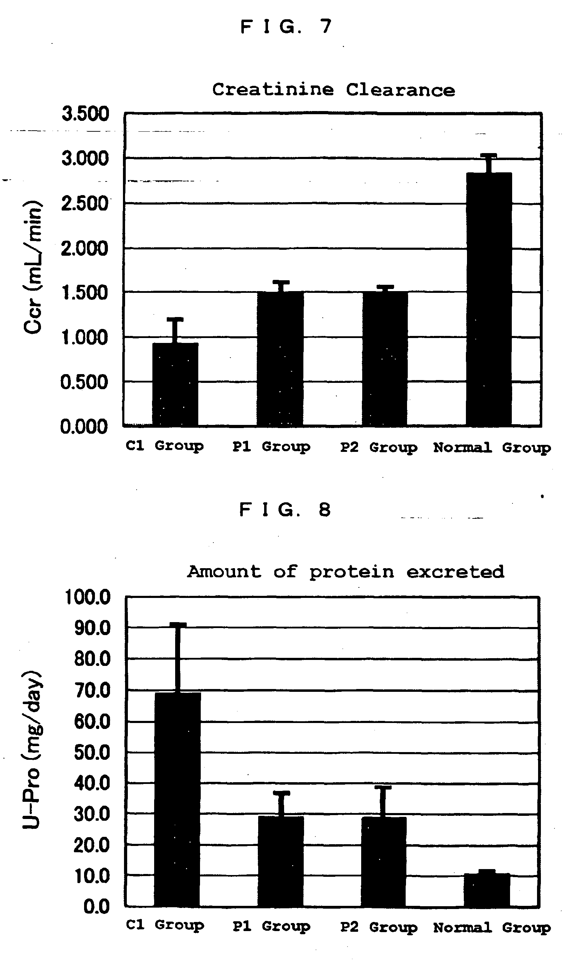

The results are shown in Figures 5 to 8. In the P1 and P2 groups, serum creatinine (Figure 5) and urea-nitrogen (Figure 6) were significantly lowered, respectively, in comparison with the C1 group, after 8 weeks from the beginning of the administration. As to creatinine clearance (Figure 7), which is an index of a renal function, a reduction was recognized in the C1 group, whereas a significant inhibition of the reduction in the C1 group was observed in the P1 and P2 groups. Further, as to the amount of protein excreted (Figure 8), an index of a function of a nephric tubules, an increase was recognized in the C1 group, whereas a significant inhibition of the increase in the C1 group was observed in the P1 and P2 groups. In addition, similar results were observed for urinary creatinine.

It is apparent from the above results that the adsorbent for oral administration of the present invention can inhibit a progress of a chronic renal failure, improve a chronic renal failure, prevent a renal hypofunction, or maintain a renal function.

Test 2 for confirming pharmacological effects: Function to improve a liver disease

Hepatitis model rats induced by carbon tetrachloride were used to carry out a test for confirming pharmacological effects on a liver disease by an administration of the adsorbent for oral administration of the present invention. The adsorbents prepared in Examples 1 and 3 according to the present invention were used as a sample.

More particularly, carbon tetrachloride was subcutaneously administered at an amount of 12 mg/kg twice a week to Sprague-Dauley rats (produced by Clea Japan, Inc.; male; 7 weeks old), continuously for about 4 months until the end of the test for confirming pharmacological effects. After two months from the beginning of the administration of carbon tetrachloride, a reduction of liver function was confirmed, and thus, the rats were divided into a control group (6 rats; hereinafter referred to as a C2 group), a group to which the adsorbent prepared in Example 1 was administered (6 rats; hereinafter referred to as a Q1 group), and a group to which the adsorbent prepared in Example 3 was administered (6 rats; hereinafter referred to as a Q2 group), so that there was no major imbalance therebetween with respect to pathosis.

A powdery feed was administered to the rats of the groups. An amount of the feed given to the rats of the groups was determined on the basis of an average amount of feed taken by the rats of the C2 group for 2 or 3 days. A mixed feed containing 5 % by weight of the adsorbent for oral administration in the same powdery feed as that administered to the C2 group was administered to the rats of the Q1 and Q2 groups for 2 months after the division to the groups. Further, a same test was carried out for six normal rats to which carbon tetrachloride was not administered (normal group).

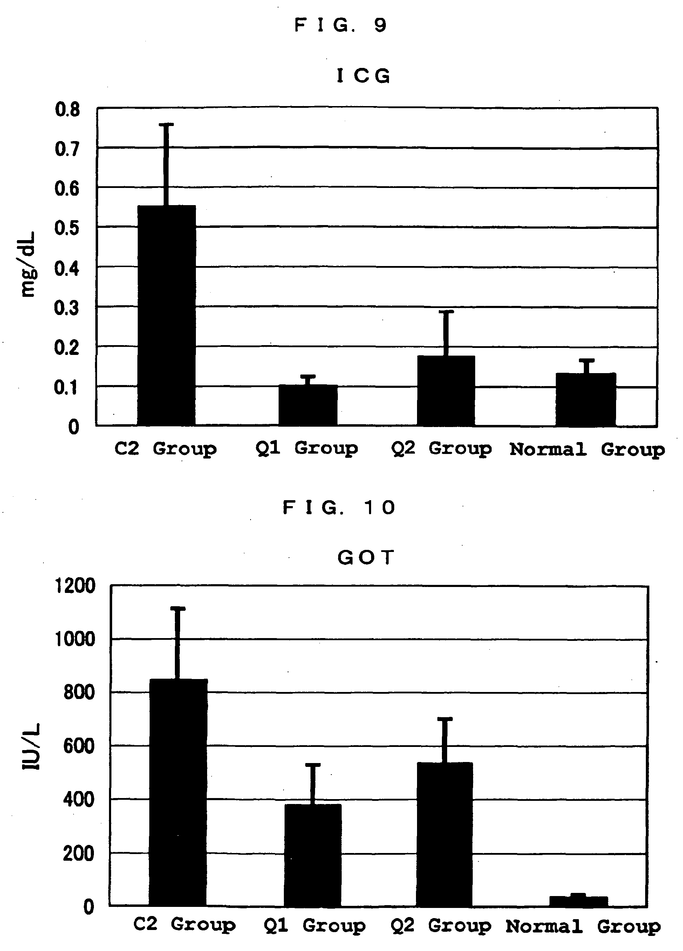

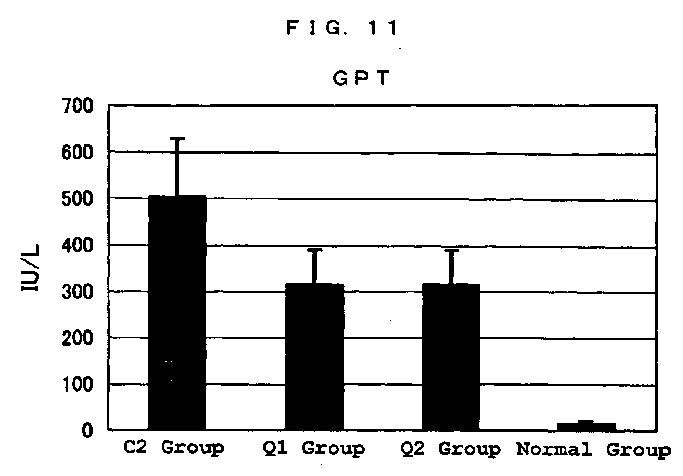

For about two months from the beginning of the administration of the adsorbent for oral administration to the end of the administration test, ICG (Indocyanine green), GOT (glutamic-oxaloacetic transaminase), and GPT (glutamic-pyruvic transaminase) were measured. The results obtained after two months from the beginning of the administration of the adsorbent for oral administration are shown in Figure 9 (ICG), Figure 10 (GOT), and Figure 11 (GPT). Comparing the ICG test reflecting hepatic mesenchymal functions, the Q1 and Q2 groups showed significantly lower values than the C2 group. Further, the Q1 and Q2 groups showed significantly lower values than the C2 group, as to GOT and GPT which are leakage of cellular enzymes.

It is apparent from the above results that the adsorbent for oral administration of the present invention can improve a deterioration of liver functions.

INDUSTRIAL APPLICABILITY

The adsorbent for oral administration according to the present invention is prepared from a thermosetting resin as a carbon source, has a specific pore structure, and thus, has an excellent selective adsorbability, that is, an excellent adsorbability of harmful toxins in an intestine, together with a low adsorbability of useful substances such as digestive enzymes or the like in a body, when orally administered, and the selective adsorbability is remarkably improved in comparison with that of the conventional adsorbent for oral administration.

The adsorbent for oral administration according to the present invention can be used as an adsorbent for oral administration for treating or preventing a renal disease, or an adsorbent for treating or preventing a liver disease.

As the renal disease, there may be mentioned, for example, chronic renal failure, acute renal failure, chronic pyelonephritis, acute pyelonephritis, chronic nephritis, acute nephritic syndrome, acute progressive nephritic syndrome, chronic nephritic syndrome, nephrotic syndrome, nephrosclerosis, interstitial nephritis, tubulopathy, lipoid nephrosis, diabetic nephropathy, renovascular hypertension, or hypertension syndrome, or secondary renal diseases caused by these primary diseases, or a light renal failure before a dialysis therapy, and may be used in an improvement of a light renal failure before a dialysis therapy or a disease condition for a patient during a dialysis therapy (see "Clinical Nephrology", Asakura-shoten, Nishio Honda, Kenkichi Koiso, and Kiyoshi Kurokawa, 1990; and "Nephrology" Igaku-shoin, Teruo Omae and Sei Fujimi, ed., 1981).

As the liver disease, there may be mentioned, for example, fulminant hepatitis, chronic hepatitis, viral hepatitis, alcoholic hepatitis, hepatic fibrosis, liver cirrhosis, hepatic cancer, autoimmune hepatitis, drug allergic hepatopathy, primary biliary cirrhosis, tremor, encephalopathia, dysbolism, or dysfunction. Further, the porous spherical carbonaceous substance can be used in a treatment of a disease caused by toxic substances in a body, such as psychosis.

Although the present invention has been described with reference to specific embodiments, various changes and modifications obvious to those skilled in the art are possible without departing from the scope of the appended claims.

**Claims**

1. An adsorbent for oral administration, comprising a spherical activated carbon prepared from a thermosetting resin as a carbon source, wherein the diameter is 0.01 to 1 mm, and the specific surface area determined by Langmuir's adsorption equation is 1000 $m^2$/g or more.

2. The adsorbent for oral administration according to claim 1, wherein the total amount of basic groups on the spherical activated carbon is 0.40 meq/g or more.

3. An adsorbent for oral administration, comprising a surface-modified spherical activated carbon prepared from a thermosetting resin as a carbon source, wherein the diameter is 0.01 to 1 mm, the specific surface area determined by Langmuir's adsorption equation is 1000 $m^2$/g or more, the total amount of acidic groups is 0.40 to 1.00 meq/g, and the total amount of basic groups is 0.40 to 1.10 meq/g.

4. An agent for treating or preventing a renal disease, comprising the adsorbent for oral administration according to any one of claims 1 to 3 as an effective component.

**5.** An agent for treating or preventing a disease bearing a relationship to or deteriorated by a uremic substance, comprising the adsorbent for oral administration according to any one of claims 1 to 3 as an effective component.

**6.** An agent for treating or preventing a liver disease, comprising the adsorbent for oral administration according to any one of claims 1 to 3 as an effective component.

**7.** A pharmaceutical composition for treating or preventing a renal disease, comprising the adsorbent for oral administration according to any one of claims 1 to 3 and a pharmaceutically acceptable carrier or diluent.

**8.** A pharmaceutical composition for treating or preventing a disease bearing a relationship to or deteriorated by a uremic substance, comprising the adsorbent for oral administration according to any one of claims 1 to 3 and a pharmaceutically acceptable carrier or diluent.

**9.** A pharmaceutical composition for treating or preventing a liver disease, comprising the adsorbent for oral administration according to any one of claims 1 to 3 and a pharmaceutically acceptable carrier or diluent.

**10.** Use of the adsorbent for oral administration according to any one of claims 1 to 3, for preparing an agent for treating or preventing a renal disease.

**11.** Use of the adsorbent for oral administration according to any one of claims 1 to 3, for preparing an agent for treating or preventing a disease bearing a relationship to or deteriorated by a uremic substance.

**12.** Use of the adsorbent for oral administration according to any one of claims 1 to 3, for preparing an agent for treating or preventing a liver disease.

**Patentansprüche**

**1.** Adsorptionsmittel für eine orale Verabreichung, das kugelförmige Aktivkohle umfasst, die aus einem wärmeaushärtbaren Kunststoff als Kohlenstoffquelle hergestellt worden ist, wobei der Durchmesser 0,01 bis 1 mm und die durch die Langmuirsche Adsorptionsgleichung bestimmte spezifische Oberfläche 1000 $m^2$/g oder mehr beträgt.

**2.** Adsorptionsmittel für eine orale Verabreichung nach Anspruch 1, worin der Gesamtanteil der basischen Gruppen an der kugelförmigen Aktivkohle 0,40 mÄqu./g oder mehr beträgt.

**3.** Adsorptionsmittel für eine orale Verabreichung, das eine oberflächenmodifizierte kugelförmige Aktivkohle umfasst, die aus einem wärmeaushärtbaren Kunststoff als Kohlenstoffquelle hergestellt ist, wobei der Durchmesser 0,01 bis 1 mm, die entsprechend der Langmuirschen Adsorptionsgleichung bestimmte spezifische Oberfläche 1000 $m^2$/g oder mehr, der Gesamtanteil der sauren Gruppen 0,40 bis 1,00 mÄqu./g und der Gesamtanteil der basischen Gruppen 0,40 bis 1,10 mÄqu./g beträgt.

**4.** Mittel zur Behandlung oder Verhütung einer Nierenerkrankung, das das Adsorptionsmittel für eine orale Verabreichung nach einem der Ansprüche 1 bis 3 als Wirkstoff umfasst.

**5.** Mittel zur Behandlung oder Verhütung einer Erkrankung, die eine Beziehung zu einer urämischen Substanz besitzt oder von dieser verschlimmert wird, welches das Adsorptionsmittel für eine orale Verabreichung nach einem der Ansprüche 1 bis 3 als Wirkstoff umfasst.

**6.** Mittel zur Behandlung oder Verhütung einer Lebererkrankung, das das Adsorptionsmittel für eine orale Verabreichung nach einem der Ansprüche 1 bis 3 als Wirkstoff umfasst.

**7.** Pharmazeutische Zusammensetzung zur Behandlung oder Verhütung einer Nierenerkrankung, die das Adsorptionsmittel für eine orale Verabreichung nach einem der Ansprüche 1 bis 3 und einen Träger oder ein Verdünnungsmittel, der/das pharmazeutisch verträglich ist, umfasst.

**8.** Pharmazeutische Zusammensetzung zur Behandlung oder Verhütung einer Erkrankung, die eine Beziehung zu einer urämischen Substanz besitzt oder von dieser verschlimmert wird, die das Adsorptionsmittel für eine orale Verabreichung nach einem der Ansprüche 1 bis 3 und einen Träger oder ein Verdünnungsmittel, der/das pharmazeutisch verträglich ist, umfasst.

9. Pharmazeutische Zusammensetzung zur Behandlung oder Verhütung einer Lebererkrankung, die das Adsorptionsmittel für orale Verabreichung nach einem der Ansprüche 1 bis 3 und einen Träger oder ein Verdünnungsmittel, der/das pharmazeutisch verträglich ist, umfasst.

10. Verwendung des Adsorptionsmittels für eine orale Verabreichung nach einem der Ansprüche 1 bis 3 zur Herstellung eines Mittels für die Behandlung oder Verhütung einer Nierenerkrankung.

11. Verwendung des Adsorptionsmittels für eine orale Verabreichung nach einem der Ansprüche 1 bis 3 zur Herstellung eines Mittels für die Behandlung oder Verhütung einer Erkrankung, die eine Beziehung zu eine urämischen Substanz besitzt oder von dieser verschlimmert wird.

12. Verwendung des Adsorptionsmittels für eine orale Verabreichung nach einem der Ansprüche 1 bis 3 zur Herstellung eines Mittels zur Behandlung oder Verhütung einer Lebererkrankung.


**Revendications**

1. Adsorbant destiné à une administration orale, comprenant du charbon actif sphérique préparé à partir d'une résine thermodurcissable comme source de carbone, dans lequel le diamètre est de 0,01 à 1 mm, et la surface spécifique déterminée par l'équation d'adsorption de Langmuir est égale ou supérieure à 1000 $m^2/g$.

2. Adsorbant destiné à une administration orale selon la revendication 1, dans lequel la quantité totale de groupes basiques sur le charbon actif sphérique est égale ou supérieure à 0,40 meq/g.

3. Adsorbant destiné à une administration orale, comprenant du charbon actif sphérique à surface modifiée, préparé à partir d'une résine thermodurcissable comme source de carbone, dans lequel le diamètre est de 0,01 à 1 mm, et la surface spécifique déterminée par l'équation d'adsorption de Langmuir est égale ou supérieure à 1000 $m^2/g$, la quantité totale de groupes acides est de 0,40 à 1,00 meq/g, et la quantité totale de groupes basiques est de 0,40 à 1,10 meq/g.

4. Agent pour traiter ou prévenir une maladie rénale, comprenant l'adsorbant destiné à une administration orale selon l'une des revendications 1 à 3, comme composant efficace.

5. Agent pour traiter ou prévenir une maladie ayant un rapport avec une substance urémique ou altérée par une substance urémique, comprenant l'adsorbant destiné à une administration orale selon l'une des revendications 1 à 3, comme composant efficace.

6. Agent pour traiter ou prévenir une maladie du foie, comprenant l'adsorbant destiné à une administration orale selon l'une des revendications 1 à 3, comme composant efficace.

7. Composition pharmaceutique pour traiter ou prévenir une maladie rénale, comprenant l'adsorbant destiné à une administration orale selon l'une des revendications 1 à 3 et un véhicule pharmaceutiquement acceptable ou un diluant.

8. Composition pharmaceutique pour traiter ou prévenir une maladie, ayant un rapport avec une substance urémique ou altérée par une substance urémique, comprenant l'adsorbant destiné à une administration orale selon l'une des revendications 1 à 3 et un véhicule pharmaceutiquement acceptable ou un diluant.

9. Composition pharmaceutique pour traiter ou prévenir une maladie du foie, comprenant l'adsorbant destiné à une administration orale selon l'une des revendications 1 à 3 et un véhicule pharmaceutiquement acceptable ou un diluant.

10. Utilisation de l'adsorbant destiné à une administration orale selon l'une des revendications 1 à 3, pour préparer un agent pour traiter ou prévenir une maladie rénale.

11. Utilisation de l'adsorbant destiné à une administration orale selon l'une des revendications 1 à 3, pour préparer un agent pour traiter ou prévenir une maladie ayant un rapport avec une substance urémique ou altérée par une substance urémique.

12. Utilisation de l'adsorbant destiné à une administration orale selon l'une des revendications 1 à 3, pour préparer un agent pour traiter ou prévenir une maladie du foie.

F I G. 1

F I G. 2

F I G. 3

F I G. 4

# F I G. 5

**Serum Creatinine**

# F I G. 6

**Urea-nitrogen in blood**

F I G. 7

Creatinine Clearance

F I G. 8

Amount of protein excreted

## F I G. 9

### I C G

## F I G. 1 0

### G O T

## F I G. 1 1

### G P T